# EUROPEAN PATENT SPECIFICATION

(11) **EP 1 152 753 B1**
(45) Date of publication and mention of the grant of the patent: **12.05.2004**
(21) Application number: 00907572.2
(22) Date of filing: 16.02.2000
(51) Int. Cl.: A61K 31/135, A61P 11/00

(54) **COMBINATIONS OF FORMOTEROL AND FLUTICASONE PROPIONATE FOR ASTHMA**
KOMBINATIONEN VON FORMOTEROL UND FLUTICASONPROPIONAT FÜR ASTHMA
COMBINAISONS DE FORMOTEROL ET DE FLUTICASONE PROPIONATE POUR LE TRAITEMENT DE L'ASTHME

(30) Priority: 18.02.1999 GB 9903759
(43) Date of publication of application: 14.11.2001
(73) Proprietor: Novartis AG, 4056 Basel (CH); Novartis Pharma GmbH, 1230 Wien (AT)
(72) Inventor: CLARKE, Jeremy, Guy, West Sussex RH13 5RP (GB); DANAHAY, Henry, Luke, Godalming, Surrey GU7 3NP (GB); HASSAN, Ian, Francis, Morris Plains, NJ 07950 (US)
(74) Representative: Grubb, Philip William
(86) International application number: PCT/EP2000/001270
(87) International publication number: WO 2000/048587

(56) References cited:
- WO-A-95/05805
- WO-A-96/19968
- WO-A-98/34595
- WO-A-98/41193
- US-A- 6 030 604
- BARNES P.J.: "Efficacy of inhaled corticosteroids in asthma." JOURNAL OF ALLERGY AND CLINICAL IMMUNOLOGY, (1998) 102/4 I (531-538). , XP000911075
- LIPWORTH B. ET AL: "Effects of treatment with formoterol on bronchoprotection against methacholine." AMERICAN JOURNAL OF MEDICINE, (1998) 104/5 (431-438). , XP000911068
- O'CONNOR B.J.: "Combination therapy." PULMONARY PHARMACOLOGY AND THERAPEUTICS, (1998) 11/5-6 (397-399). , XP000911059

## Description

This invention relates to combinations of a beta-2 agonist and a steroid and their use for the treatment of inflammatory or obstructive airways diseases.

Formoterol,N-[2-hydroxy-5-(1-hydroxy-2-((2-(4-methoxyphenyl)-1-methylethyl)amino)-ethyl)phenyl]formamide, particularly in the form of its fumarate salt, is a bronchodilator used in the treatment of inflammatory or obstructive airways diseases. Fluticasone propionate, S-fluoromethyl 6α,9α-difluoro-11β-hydroxy-16α-methyl-3-oxo-17α-propionyloxyandrosta-1,4-diene-17β-carbothioate, an anti-inflammatory corticosteroid, is described in US4335121.

It has now surprisingly been found that a significant unexpected therapeutic benefit, particularly a synergistic therapeutic benefit, in the treatment of inflammatory or obstructive airways diseases can be obtained by using a composition containing formoterol, or a salt or solvate thereof, and fluticasone propionate. For instance, it is possible using such a composition to reduce the dosages of fluticasone propionate required for a given therapeutic effect considerably compared with those required using treatment with fluticasone propionate alone, thereby minimising possibly undesirable side effects. In particular, it has been found that compositions containing formoterol and fluticasone propionate induce an anti-inflammatory activity which is significantly greater than that induced by formoterol or fluticasone propionate alone and that the amount of fluticasone propionate needed for a given anti-inflammatory effect may be significantly reduced when used in admixture with formoterol, thereby reducing the risk of undesirable side effects from the repeated exposure to the steroid involved in the treatment of inflammatory or obstructive airways diseases.

Furthermore, using the compositions of the invention, medicaments which have a rapid onset of action and a long duration of action may be prepared. Moreover, with the compositions of the invention, medicaments which result in a significant improvement in lung function may be prepared. In another aspect, with the compositions of the invention, medicaments which provide improved control of obstructive or inflammatory airways diseases, or a reduction in exacerbations of such diseases, may be prepared. In a further aspect, with the compositions of the invention, medicaments in rescue treatment of obstructive or inflammatory airways diseases, or which reduce or eliminate the need for treatment with short-acting rescue medicaments such as salbutamol or terbutaline, may be prepared; thus medicaments based on compositions of the invention facilitate the treatment of an obstructive or inflammatory airways disease with a single medicament.

Accordingly, in one aspect, the present invention provides a pharmaceutical composition comprising (A) formoterol or a pharmaceutically acceptable salt thereof or a solvate of formoterol or said salt and (B) fluticasone propionate.

In a further aspect, the present invention provides a phamaceutical composition comprising a mixture of effective amounts of (A) and (B) as hereinbefore defined together with a pharmaceutically acceptable carrier.

In a yet further aspect, the present invention provides a pharmaceutical composition for use in the treatment of an inflammatory or obstructive airways disease comprising (A) and (B) as hereinbefore defined.

The present invention still further provides the use of a pharmaceutical composition comprising (A) and (B) as hereinbefore defined for the preparation of a medicament for the treatment of an inflammatory or obstructive airways disease.

Pharmaceutically acceptable salts of formoterol include, for example, salts of inorganic acids such as hydrochloric, hydrobromic, sulfuric and phosphoric acids, and organic acids such as fumaric, maleic, acetic, lactic, citric, tartaric, ascorbic, succinic, glutaric, gluconic, tricarballylic, oleic, benzoic, p-methoxybenzoic, salicylic, o- and p-hydroxybenzoic, p-chlorobenzoic, methanesulfonic, p-toluenesulfonic and 3-hydroxy-2-naphthalene carboxylic acids.

Component (A) may be in any isomeric form or mixture of isomeric forms, for example a pure enantiomer, a mixture of enantiomers, a racemate or a mixture thereof. It may be in the form of a solvate, for example a hydrate, thereof, for example as described in US3994974 or US5684199, and may be present in a particular crystalline form, for example as described in WO95/05805. Preferably, component (A) is formoterol fumarate, especially in the form of the dihydrate.

Administration of the pharmaceutical composition as hereinbefore described is preferably by inhalation, in which case (A) and (B) are in inhalable form. The inhalable form of the composition may be, for example, an atomizable composition such as an aerosol comprising the active ingredients, i.e. (A) and (B), in solution or dispersion in a propellant, or a nebulizable composition comprising a dispersion of the active ingredients in an aqueous, organic or aqueous/organic medium. For example, the inhalable form of the pharmaceutical composition may be an aerosol comprising a mixture of (A) and (B) in solution or dispersion in a propellant. In another example, the inhalable form is a nebulizable composition comprising a dispersion of (A) and (B) in an aqueous, organic or aqueous/organic medium.

An aerosol composition suitable for use as the inhalable form of the composition of the invention may comprise the active ingredients in solution or dispersion in a propellant, which may be chosen from any of the propellants known in the art. Suitable such propellants include hydrocarbons such as n-propane, n-butane or isobutane or mixtures of two or more such hydrocarbons, and halogen-substituted hydrocarbons, for example fluorine-substituted methanes, ethanes, propanes, butanes, cyclopropanes or cyclobutanes, particularly 1,1,1,2-tetrafluoroethane (HFA134a) and 1,1,1,2,3,3,3-heptafluoropropane (HFA227), or mixtures of two or more such halogen-substituted hydrocarbons. Where (A) and/or (B) are present in suspension in the propellant, i.e. where present in particulate form dispersed in the propellant, the aerosol composition may also contain a lubricant and a surfactant, which may be chosen from those lubricants and surfactants known in the art. Other suitable aerosol compositions include surfactant-free or substantially surfactant-free aerosol compositions. The aerosol composition may contain up to about 5% by weight, for example 0.002 to 5%, 0.01 to 3%, 0.015 to 2 %, 0.1 to 2%, 0.5 to 2 % or 0.5 to 1%, by weight of the mixture of (A) and (B), based on the weight of the propellant. Where present, the lubricant and surfactant may be in an amount up to 5% and 0.5% respectively by weight of the aerosol composition. The aerosol composition may also contain a co-solvent such as ethanol in an amount up to 30% by weight of the composition, particularly for administration from a pressurised metered dose inhalation device.

In another embodiment of the invention, the inhalable form is a dry powder, i.e. (A) and (B) are present in a dry powder comprising finely divided (A) and (B) optionally together with a finely divided pharmaceutically acceptable carrier, which is preferably present and may be one or more materials chosen from materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran or mannitol. An especially preferred carrier is lactose, particularly in the form of the monohydrate. The dry powder may be in capsules of gelatin or plastic, or in blisters, for use in a dry powder inhalation device, preferably in dosage units of the mixture of (A) and (B) together with the carrier in amounts to bring the total weight of powder in each capsule to from S mg to 50 mg. Alternatively, the dry powder may be contained in a reservoir of a multi-dose dry powder inhalation device.

In the finely divided particulate form of the composition of the invention, (A) and (B) may each have an average particle diameter of up to about 10 µm, for example 0.1 to 5 µm, preferably 1 to 5 µm. In the aerosol composition where (A) and/or (B) are present in particulate form, (A) and/or (B) may have an average particle diameter of up to about 10 µm, for example 0.1 to 5 µm, preferably 1 to 5 µm. The solid carrier, where present, generally has a maximum particle diameter of 300 µm, preferably 212 µm, and conveniently has a mean particle diameter of 40 to 100 µm, preferably 50 to 75 µm. The particle size of the active ingredients (A) and (B), and that of a solid carrier where present in dry powder compositions, can be reduced to the desired level by conventional methods, for example by grinding in an air-jet mill, ball mill or vibrator mill, microprecipitation, spray-drying, lyophilisation or recrystallisation from supercritical media.

The inhalable pharmaceutical composition of the invention may be administered using an inhalation device suitable for the inhalable form, such devices being well known in the art. Accordingly, the invention also provides a pharmaceutical product comprising a pharmaceutical composition comprising (A) and (B) as hereinbefore described in inhalable form as hereinbefore described in association with one or more inhalation devices. In a further aspect, the invention provides an inhalation device containing a pharmaceutical composition comprising (A) and (B) as hereinbefore described in inhalable form as hereinbefore described.

Where the inhalable form of the composition of the invention is an aerosol composition, the inhalation device may be an aerosol vial provided with a valve adapted to deliver a metered dose, such as 10 to 100 µl, e.g. 25 to 50 µl, of the composition, i.e. a device known as a metered dose inhaler. Suitable such aerosol vials and procedures for containing within them aerosol compositions under pressure are well known to those skilled in the art of inhalation therapy. For example, an aerosol composition may be administered from a coated can, for example as described in EP-A-0642992. Where the inhalable form of the composition of the invention is a nebulizable aqueous, organic or aqueous/organic dispersion, the inhalation device may be a known nebulizer, for example a conventional pneumatic nebulizer such as an airjet nebulizer, or an ultrasonic nebulizer, which may contain, for example, from 1 to 50 ml, commonly 1 to 10 ml, of the dispersion; or a hand-held nebulizer, for example an electronically controlled device such as an AERx (ex Aradigm, US) or a mechanical device such as a RESPIMAT (Boehringer Ingelheim) nebulizer which allows much smaller nebulized volumes, e.g. 10 to 100 µl, than conventional nebulizers. Where the inhalable form of the composition of the invention is the finely divided particulate form, the inhalation device may be, for example, a dry powder inhalation device adapted to deliver dry powder from a capsule or blister containing a dosage unit of the dry powder or a multidose dry powder inhalation (MDPI) device adapted to deliver, for example, 5-25 mg of dry powder per actuation. Suitable such dry powder inhalation devices are well known. For example, a suitable device for delivery of dry powder in encapsulated form is that described in US3991761, while a suitable MDPI device is that described in WO97/20589.

The weight ratio of formoterol, or salt or solvate thereof, to fluticasone propionate may be, in general, from 3:1 to 1:3000, for example from 2:1 to 1:2000, from 1:1 to 1: 1000, from 1:2 to 1:500 or from 1:5 to 1:50. More usually, this ratio is from 1:10 to 1 to 1:25, for example from 1:10 to 1:20. Specific examples of this ratio, to the nearest whole number, include 1:10, 1:11, 1:12, 1:13, 1:14, 1:15, 1:16, 1:17, 1:18, 1:19, 1:20, 1:21, 1:22, 1:23, 1:24 and 1:25. The above weight ratios apply particularly where (A) is formoterol fumarate dihydrate. Thus, since the molecular weights of formoterol fumarate dihydrate and fluticasone propionate are 840.9 and 500.6 respectively, the corresponding molar ratios of (A) to (B) may be, in general, from 1.79:1 to 1:5017, for example from 1.2:1 to 1:3345, from 0.6:1 to 1:1672, from 1:3.34 to 1:836 or from 1:8.36 to 1:83.6; more usually from 1:16.7 to 1:41.8, for example from 1:16.7 to 1:33.4; specific examples of the molar ratio being 1:16.7, 1:18.4, 1:20.1, 1:21.7, 1:23.4, 1:25.1, 1:26.8, 1:28.4, 1:30.1, 1:31.8, 1:33.4, 1:35.1, 1:36.8, 1:38.5, 1:40.1, and 1:41.8.

A suitable daily dose of formoterol, or salt or solvate thereof, particularly as formoterol fumarate dihydrate, for inhalation in a composition of the invention may be from 1 to 72 pg, for example from 1 to 60 pg, generally from 3 to 50 pg, preferably from 6 to 48 pg, for instance from 6 to 24 pg. A suitable daily dose of fluticasone propionate for inhalation in a composition of the invention may be from 25 to 3000 µg, for example from 25 to 2000µg, from 50 to 2000µg, preferably from 100 to 1000 µg, for instance from 200 to 1000 pg or from 200 to 500 µg .The precise dose used will of course depend on the condition to be treated, the patient and the efficiency of the inhalation device. The formulation of a composition of the invention and its frequency of administration may be chosen accordingly. A suitable unit dose of formoterol component (A), particularly as formoterol fumarate dihydrate, in a composition of the invention may be from 1 to 72 µg, for example from 1 to 60 µg, generally from 3 to 48 µg, preferably from 6 to 36 µg, especially from 12 to 24 µg. A suitable unit dose of fluticasone propionate (B) in a composition of the invention may be from 25 µg to 500 µg, for example from 50 µg to 400 µg, preferably from 100 µg to 300 µg, especially from 150 to 250 µg. These unit doses may suitably be administered once or twice daily in accordance with the suitable daily dose mentioned hereinbefore. For on demand usage, a dosage unit containing 6µg or 12 µg of (A) and 50 µg or 100µg of fluticasone propionate (B) is preferred.

In one preferred embodiment of the invention, when the pharmaceutical composition of the invention is a dry powder in a capsule containing a unit dose of (A) and (B), for example for inhalation from a single capsule inhaler, the capsule may suitably contain, where (A) is formoterol fumarate dihydrate, from 3 µg to 36 µg of (A), preferably from 6 µg to 24 µg of (A), especially from 12 µg to 24 µg of (A), and from 25 µg to 500 µg of (B), preferably from 50 µg to 250 µg of (B), especially from 100 to 250 µg of (B), together with a pharmaceutically acceptable carrier as hereinbefore described in an amount to bring the total weight of dry powder per capsule to between 5 mg and 50mg, for example 5mg, 10mg, 15mg, 20mg, 25mg, 30mg, 35mg, 40mg, 45mg or 50mg, preferably 20 to 25 mg, especially 25 mg.

In another preferred embodiment of the invention, the pharmaceutical composition of the invention is a dry powder for administration from a reservoir of a multi-dose dry powder inhaler adapted to deliver 3mg to 25mg of powder containing a unit dose of (A) and (B) per actuation, for example, where (A) is formoterol fumarate dihydrate, a powder comprising, by weight, 3 to 36 parts, preferably 6 to 24 parts, especially 12 to 24 parts of (A); 25 to 500 parts, preferably 50 to 400 parts, especially 100 to 250 parts of (B); and 2464 to 24972 parts, preferably 4464 to 14972 parts, especially 4464 to 9972 parts of a pharmaceutically acceptable carrier as hereinbefore described.

Treatment of inflammatory or obstructive airways diseases in accordance with the invention may be symptomatic or prophylactic treatment. Inflammatory or obstructive airways diseases to which the present invention is applicable include asthma of whatever type or genesis including both intrinsic (non-allergic) asthma and extrinsic (allergic) asthma. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant form any previously administered symptomatic asthma therapy.

Other inflammatory or obstructive airways diseases and conditions to which the present invention is applicable include acute lung injury (ALI), acute respiratory distress syndrome (ARDS), chronic obstructive pulmonary, airways or lung disease (COPD, COAD or COLD), including chronic bronchitis and emphysema, bronchiectasis and exacerbation of airways hyperreactivity consequent to other drug therapy, in particular other inhaled drug therapy. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

The invention is illustrated by the following Examples, in which parts are by weight unless stated otherwise.

### Example 1 - Aerosol Composition for Metered Dose Inhaler

| Ingredient | % by weight |
|---|---|
| Formoterol fumarate dihydrate | 0.012 |
| Fluticasone propionate | 0.250 |
| Ethanol (absolute) | 2.500 |
| HFA 227 | 60.768 |
| HFA134a | 36.470 |

### Example 2 - Dry Powder

| Ingredient | % by weight |
|---|---|
| Formoterol fumarate dihydrate | 0.048 |
| Fluticasone propionate | 1.000 |
| Lactose monohydrate | 98.952 |

### Example 3

A dry powder suitable for delivery from the reservoir of the multi-dose inhaler described in WO97/20589 is prepared by mixing 12 parts of formoterol fumarate dihydrate which has been ground to a mean particle diameter of 1-5µm in an air-jet mill, 250 parts of fluticasone propionate which has been similarly ground to a mean particle diameter of 1-5µm and 4738 parts of lactose monohydrate having a particle diameter below 212µm.

### Examples 4 - 92

Example 3 is repeated, but using the amounts of the ingredients shown in the table below in place of the amounts used in that Example :

| Example | Formoterol Fumarate Dihydrate (Parts) | Fluticasone Propionate (Parts) | Lactose Monohydrate (Parts) |
|---|---|---|---|
| 4 | 12 | 50 | 4938 |
| 5 | 12 | 100 | 4888 |
| 6 | 12 | 150 | 4838 |
| 7 | 12 | 200 | 4788 |
| 8 | 6 | 50 | 4944 |
| 9 | 6 | 100 | 4894 |
| 10 | 6 | 150 | 4844 |
| 11 | 6 | 200 | 4794 |
| 12 | 6 | 250 | 4744 |
| 13 | 18 | 50 | 4932 |
| 14 | 18 | 100 | 4882 |
| 15 | 18 | 150 | 4832 |
| 16 | 18 | 200 | 4782 |
| 17 | 18 | 250 | 4732 |
| 18 | 24 | 50 | 4926 |
| 19 | 24 | 100 | 4876 |
| 20 | 24 | 150 | 4826 |
| 21 | 24 | 200 | 4776 |
| 22 | 24 | 250 | 4726 |
| 23 | 30 | 50 | 4920 |
| 24 | 30 | 100 | 4870 |
| 25 | 30 | 150 | 4820 |
| 26 | 30 | 200 | 4770 |
| 27 | 30 | 250 | 4720 |
| 28 | 36 | 50 | 4914 |
| 29 | 36 | 100 | 4864 |
| 30 | 36 | 150 | 4814 |
| 31 | 36 | 200 | 4764 |
| 32 | 36 | 250 | 4714 |
| 33 | 6 | 50 | 9944 |
| 34 | 6 | 100 | 9894 |
| 35 | 6 | 150 | 9844 |
| 36 | 6 | 200 | 9794 |
| 37 | 6 | 250 | 9744 |
| 38 | 12 | 50 | 9938 |
| 39 | 12 | 100 | 9888 |
| 40 | 12 | 150 | 9838 |
| 41 | 12 | 200 | 9788 |
| 42 | 12 | 250 | 9738 |
| 43 | 18 | 50 | 9932 |
| 44 | 18 | 100 | 9882 |
| 45 | 18 | 150 | 9832 |
| 46 | 18 | 200 | 9782 |
| 47 | 18 | 250 | 9732 |
| 48 | 24 | 50 | 9926 |
| 49 | 24 | 100 | 9876 |
| 50 | 24 | 150 | 9826 |
| 51 | 24 | 200 | 9776 |
| 52 | 24 | 250 | 9726 |
| 53 | 30 | 50 | 9920 |
| 54 | 30 | 100 | 9870 |
| 55 | 30 | 150 | 9820 |
| 56 | 30 | 200 | 9770 |
| 57 | 30 | 250 | 9720 |
| 58 | 36 | 50 | 9914 |
| 59 | 36 | 100 | 9864 |
| 60 | 36 | 150 | 9814 |
| 61 | 36 | 200 | 9764 |
| 62 | 36 | 250 | 9714 |
| 63 | 6 | 50 | 14944 |
| 64 | 6 | 100 | 14894 |
| 65 | 6 | 150 | 14844 |
| 66 | 6 | 200 | 14794 |
| 67 | 6 | 250 | 14744 |
| 68 | 12 | 50 | 14938 |
| 69 | 12 | 100 | 14888 |
| 70 | 12 | 150 | 14838 |
| 71 | 12 | 200 | 14788 |
| 72 | 12 | 250 | 14738 |
| 73 | 18 | 50 | 14932 |
| 74 | 18 | 100 | 14882 |
| 75 | 18 | 150 | 14832 |
| 76 | 18 | 200 | 14782 |
| 77 | 18 | 250 | 14732 |
| 78 | 24 | 50 | 14926 |
| 79 | 24 | 100 | 14876 |
| 80 | 24 | 150 | 14826 |
| 81 | 24 | 200 | 14776 |
| 82 | 24 | 250 | 14726 |
| 83 | 30 | 50 | 14920 |
| 84 | 30 | 100 | 14870 |
| 85 | 30 | 150 | 14820 |
| 86 | 30 | 200 | 14770 |
| 87 | 30 | 250 | 14720 |
| 88 | 36 | 50 | 14914 |
| 89 | 36 | 100 | 14864 |
| 90 | 36 | 150 | 14814 |
| 91 | 36 | 200 | 14764 |
| 92 | 36 | 250 | 14714 |

### Example 93

Gelatin capsules suitable for use in a capsule inhaler such as that described in US3991761 are prepared, each capsule containing a dry powder obtained by mixing 12µg of formoterol fumarate dihydrate which has been ground to a mean particle diameter of 1 to 5µm in an air jet mill, 250µg of fluticasone propionate which has been similarly ground to a mean particle diameter of 1 to 5µm and 24738µg of lactose monohydrate having a particle diameter below 212µm.

### Examples 94 - 152

Example 93 is repeated, but using the amounts of the ingredients shown in the table below in place of the amounts used in that Example :

| Example | Formoterol Fumarate Dihydrate (Parts) | Fluticasone Propionate (Parts) | Lactose Monohydrate (Parts) |
|---|---|---|---|
| 94 | 12 | 50 | 24938 |
| 95 | 12 | 100 | 24888 |
| 96 | 12 | 150 | 24838 |
| 97 | 12 | 200 | 24788 |
| 98 | 6 | 50 | 24944 |
| 99 | 6 | 100 | 24894 |
| 100 | 6 | 150 | 24844 |
| 101 | 6 | 200 | 24794 |
| 102 | 6 | 250 | 24744 |
| 103 | 18 | 50 | 24932 |
| 104 | 18 | 100 | 24882 |
| 105 | 18 | 150 | 24832 |
| 106 | 18 | 200 | 24782 |
| 107 | 18 | 250 | 24732 |
| 108 | 24 | 50 | 24926 |
| 109 | 24 | 100 | 24876 |
| 110 | 24 | 150 | 24826 |
| 111 | 24 | 200 | 24776 |
| 112 | 24 | 250 | 24726 |
| 113 | 30 | 50 | 24920 |
| 114 | 30 | 100 | 24870 |
| 115 | 30 | 150 | 24820 |
| 116 | 30 | 200 | 24770 |
| 117 | 30 | 250 | 24720 |
| 118 | 36 | 50 | 24914 |
| 119 | 36 | 100 | 24864 |
| 120 | 36 | 150 | 24814 |
| 121 | 36 | 200 | 24764 |
| 122 | 36 | 250 | 24714 |
| 123 | 6 | 50 | 19944 |
| 124 | 6 | 100 | 19894 |
| 125 | 6 | 150 | 19844 |
| 126 | 6 | 200 | 19794 |
| 127 | 6 | 250 | 19744 |
| 128 | 12 | 50 | 19938 |
| 129 | 12 | 100 | 19888 |
| 130 | 12 | 150 | 19838 |
| 131 | 12 | 200 | 19788 |
| 132 | 12 | 250 | 19738 |
| 133 | 18 | 50 | 19932 |
| 134 | 18 | 100 | 19882 |
| 135 | 18 | 150 | 19832 |
| 136 | 18 | 200 | 19782 |
| 137 | 18 | 250 | 19732 |
| 138 | 24 | 50 | 19926 |
| 139 | 24 | 100 | 19876 |
| 140 | 24 | 150 | 19826 |
| 141 | 24 | 200 | 19776 |
| 142 | 24 | 250 | 19726 |
| 143 | 30 | 50 | 19920 |
| 144 | 30 | 100 | 19870 |
| 145 | 30 | 150 | 19820 |
| 146 | 30 | 200 | 19770 |
| 147 | 30 | 250 | 19720 |
| 148 | 36 | 50 | 19914 |
| 149 | 36 | 100 | 19864 |
| 150 | 36 | 150 | 19814 |
| 151 | 36 | 200 | 19764 |
| 152 | 36 | 250 | 19714 |

### Examples 153 - 176

Example 3 is repeated, but using the amounts of the ingredients shown in the table below in place of the amounts used in that Example:

| Example | Formoterol Fumarate Dihydrate (Parts) | Fluticasone Propionate (Parts) | Lactose Monohydrate (Parts) |
|---|---|---|---|
| 153 | 6 | 25 | 2969 |
| 154 | 6 | 50 | 2944 |
| 155 | 6 | 100 | 2894 |
| 156 | 6 | 150 | 2844 |
| 157 | 6 | 200 | 2794 |
| 158 | 6 - | 250 | 2744 |
| 159 | 12 | 25 | 2963 |
| 160 | 12 | 50 | 2938 |
| 161 | 12 | 100 | 2888 |
| 162 | 12 | 150 | 2838 |
| 163 | 12 | 200 | 2788 |
| 164 | 12 | 250 | 2738 |
| 165 | 12 | 300 | 2638 |
| 166 | 12 | 350 | 2588 |
| 167 | 12 | 400 | 2538 |
| 168 | 24 | 25 | 2951 |
| 169 | 24 | 50 | 2926 |
| 170 | 24 | 100 | 2876 |
| 171 | 24 | 150 | 2826 |
| 172 | 24 | 200 | 2776 |
| 173 | 24 | 250 | 2726 |
| 174 | 24 | 300 | 2676 |
| 175 | 24 | 350 | 2626 |
| 176 | 24 | 400 | 2576 |

### Examples 177-216

Example 93 is repeated, but using the amounts of the ingredients shown in the table below in place of the amounts used in that Example:

| Example | Formoterol Fumarate Dihydrate (µg) | Fluticasone Propionate (µg) | Lactose Monohydrate (µg) |
|---|---|---|---|
| 177 | 6 | 25 | 14969 |
| 178 | 6 | 50 | 14944 |
| 179 | 6 | 100 | 14894 |
| 180 | 6 | 150 | 14844 |
| 181 | 6 | 200 | 14794 |
| 182 | 6 | 250 | 14744 |
| 183 | 6 | 300 | 14694 |
| 184 | 6 | 350 | 14644 |
| 185 | 6 | 400 | 14594 |
| 186 | 12 | 25 | 14963 |
| 187 | 12 | 50 | 14938 |
| 188 | 12 | 100 | 14888 |
| 189 | 12 | 150 | 14838 |
| 190 | 12 | 200 | 14788 |
| 191 | 12 | 250 | 14738 |
| 192 | 12 | 300 | 14688 |
| 193 | 12 | 350 | 14638 |
| 194 | 12 | 400 | 14588 |
| 195 | 12 | 500 | 14488 |
| 196 | 24 | 25 | 14951 |
| 197 | 24 | 50 | 14926 |
| 198 | 24 | 100 | 14876 |
| 199 | 24 | 150 | 14826 |
| 200 | 24 | 200 | 13876 |
| 201 | 24 | 250 | 13826 |
| 202 | 24 | 300 | 13776 |
| 203 | 6 | 25 | 9969 |
| 204 | 6 | 50 | 9944 |
| 205 | 6 | 100 | 9894 |
| 206 | 6 | 150 | 9844 |
| 207 | 6 | 200 | 9794 |
| 208 | 6 | 250 | 9744 |
| 209 | 6 | 300 | 9694 |
| 210 | 12 | 25 | 9963 |
| 211 | 12 | 50 | 9938 |
| 212 | 12 | 100 | 9888 |
| 213 | 12 | 150 | 9838 |
| 214 | 12 | 200 | 9788 |
| 215 | 12 | 250 | 9738 |
| 216 | 12 | 300 | 9688 |

## Claims

1. A pharmaceutical composition comprising (A) formoterol or a pharmaceutically acceptable salt thereof or a solvate of formoterol or said salt and (B) fluticasone propionate.

2. A composition according to claim 1 comprising a mixture of effective amounts of (A) and (B) together with a pharmaceutically acceptable carrier.

3. A composition according to claim 1 or 2, in which (A) is formoterol fumarate dihydrate.

4. A composition according to any one of the preceding claims, which is in inhalable form.

5. A composition according to claim 4, which is
(i) an aerosol comprising a mixture of (A) and (B) in solution or dispersion in a propellant; or
(ii) a nebulizable composition comprising a dispersion of (A) and (B) in an aqueous, organic or aqueous/organic medium; or
(iii) a dry powder comprising finely divided (A) and (B) optionally together with a pharmaceutically acceptable carrier in finely divided form.

6. A composition according to claim 5, which is said aerosol in which (A) and (B) are in suspension in a halogen-substituted hydrocarbon propellant.

7. A composition according to claim 5 which is said dry powder, in which the carrier is present and is a saccharide.

8. A composition according to claim 6 or 7, which (A) and/or (B) has an average particle diameter of up to 10 µm.

9. A composition according to any one of claims 1 to 8, in which the weight ratio of (A) to (B) is from 1:5 to 1:50.

10. A composition according to claim 1, which is a dry powder in a capsule, the capsule containing from 3 to 36 µg of (A) as formoterol fumarate dihydrate, from 25 to 500 µg of (B) and a pharmaceutically acceptable carrier in an amount to bring the total weight of dry powder to between 5 mg and 50 mg.

11. A composition according to claim 1, which is a dry powder comprising, by weight, 3 to 36 parts of (A) as formoterol fumarate dihydrate, 25 to 500 parts of (B) and 2464 to 24972 parts of a pharmaceutically acceptable carrier.

12. The use of a composition according to any one of claims 1 to 11 for the preparation of a medicament for the treatment of an inflammatory or obstructive airways disease.

## Patentansprüche

1. Pharmazeutische Zusammensetzung enthaltend (A) Formoterol oder ein pharmazeutisch annehmbares Salz hiervon oder ein Solvat von Formoterol oder ein solches Salz hiervon und (B) Fluticasonpropionat.

2. Zusammensetzung nach Anspruch 1 enthaltend ein Gemisch aus wirksamen Mengen von (A) und (B) zusammen mit einem pharmazeutisch annehmbaren Träger.

3. Zusammensetzung nach Anspruch 1 oder 2, worin (A) Formoterolfumaratdihydrat ist.

4. Zusammensetzung nach einem der vorhergehenden Ansprüche, die in inhalierbarer Form vorliegt.

5. Zusammensetzung nach Anspruch 4, welche folgendes ist
(i) ein Aerosol enthaltend ein Gemisch aus (A) und (B) in Lösung oder Dispersion in einem Treibmittel oder
(ii) eine vernebelbare Zusammensetzung enthaltend eine Dispersion aus (A) und (B) in einem wässrigen, organischen oder wässrigen/organischen Medium oder
(iii) ein Trockenpulver enthaltend feinverteiltes (A) und (B) gegebenenfalls zusammen mit einem pharmazeutisch annehmbaren Träger in feinverteilter Form.

6. Zusammensetzung nach Anspruch 5, welche ein Aerosol ist, worin (A) und (B) in Suspension in einem halogensubstituierten Kohlenwasserstofftreibmittel vorliegen.

7. Zusammensetzung nach Anspruch 5, welche ein trockenes Pulver ist, worin der Träger vorhanden und ein Saccharid ist.

8. Zusammensetzung nach Anspruch 6 oder 7, bei welcher (A) und/oder (B) einen mittleren Teilchendurchmesser von bis zu 10 µm haben.

9. Zusammensetzung nach einem der Ansprüche 1 bis 8, bei welchem das Gewichtsverhältnis von (A) zu (B) von 1:5 bis 1:50 reicht.

10. Zusammensetzung nach Anspruch 1, welche ein trockenes Pulver in einer Kapsel ist, die 3 bis 36 µg (A) als Formoterolfumaratdihydrat, 25 bis 500 µg (B) und einen pharmazeutisch annehmbaren Träger in einer Menge enthält, dass hierdurch das Gesamtgewicht an trockenem Pulver auf 5 mg bis 50 mg gebracht wird.

11. Zusammensetzung nach Anspruch 1, welche ein trockenes Pulver ist, das 3 bis 36 Gew.-Teile (A) als Formoterolfumaratdihydrat, 25 bis 500 Gew.-Teile (B) und 2464 bis 24972 Gew.-Teile eines pharmazeutisch annehmbaren Trägers enthält.

12. Verwendung einer Zusammensetzung nach einem der Ansprüche 1 bis 11 zur Herstellung eines Arzneimittels für die Behandlung einer entzündlichen oder obstruktiven Atemwegerkrankung.

## Revendications

1. Composition pharmaceutique comprenant (A) du formotérol ou un de ses sels pharmaceutiquement acceptables, ou un produit de solvatation de formotérol ou dudit sel, et (B) du propionate de fluticasone.

2. Composition suivant la revendication 1, comprenant un mélange de quantités efficaces de (A) et de (B), conjointement avec un support pharmaceutiquement acceptable.

3. Composition suivant la revendication 1 ou 2, dans laquelle (A) est le fumarate de formotérol dihydraté.

4. Composition suivant l'une des revendications précédentes, qui est sous forme inhalable.

5. Composition suivant la revendication 4, qui est
(i) un aérosol comprenant un mélange de (A) et de (B) en solution ou en dispersion dans un gaz propulseur ; ou
(ii) un aérosol comprenant une dispersion de (A) et de (B) dans un milieu aqueux, organique ou aqueux/organique ; ou
(iii) une poudre sèche comprenant (A) et (B) finement divisés, facultativement conjointement avec un support pharmaceutiquement acceptable sous forme finement divisée.

6. Composition suivant la revendication 5, qui est ledit aérosol dans lequel (A) et (B) sont en suspension dans un gaz propulseur hydrocarboné à substitution halogène.

7. Composition suivant la revendication 5, qui est ladite poudre sèche dans laquelle le support est présent, et est un saccharide.

8. Composition suivant la revendication 6 ou 7, dans laquelle (A) et/ou (B) a(ont) un diamètre particulaire moyen pouvant atteindre 10 µm.

9. Composition suivant l'une des revendications 1 à 8, dans laquelle le rapport pondéral de (A) à (B) est de 1:5 à 1:50.

10. Composition suivant la revendication 1, qui est une poudre sèche dans une capsule, la capsule contenant 3 à 36 µg de (A) sous forme de fumarate de formotérol dihydraté, 25 à 500 µg de (B), et un support pharmaceutiquement acceptable en une quantité permettant de porter le poids total de la poudre sèche dans l'intervalle de 5 mg à 50 mg.

11. Composition suivant la revendication 1, qui est une poudre sèche comprenant, en poids : 3 à 36 parties de (A) sous forme de fumarate de formotérol dihydraté, 25 à 500 parties de (B) et 2464 à 24972 parties d'un support pharmaceutiquement acceptable.

12. Utilisation d'une composition suivant l'une des revendications 1 à 11, pour la préparation d'un médicament pour le traitement d'une maladie inflammatoire ou obstructrice des voies aériennes.
